(19) Europäisches Patentamt
European Patent Office
Office européen des brevets

(11) Veröffentlichungsnummer: **0 022 748**
**A1**

(12) # EUROPÄISCHE PATENTANMELDUNG

(21) Anmeldenummer: **80810220.6**

(22) Anmeldetag: **07.07.80**

(51) Int. Cl.³: **A 01 N 43/40**, C 07 D 213/70

(30) Priorität: **11.07.79 CH 6476/79**

(43) Veröffentlichungstag der Anmeldung: **21.01.81 Patentblatt 81/3**

(84) Benannte Vertragsstaaten: **AT BE CH DE FR GB IT LI NL**

(71) Anmelder: **CIBA-GEIGY AG, Patentabteilung Postfach, CH-4002 Basel (CH)**

(72) Erfinder: **Ehrenfreund, Josef, Dr., Langenhagweg 11, CH-4123 Allschwil (CH)**

(54) **Pyridinderivate, Verfahren zu ihrer Herstellung, diese Verbindungen enthaltende Mittel und ihre Verwendung zur Bekämpfung von Schädlingen.**

(57) Propargylthio-nitropyridine der Formel

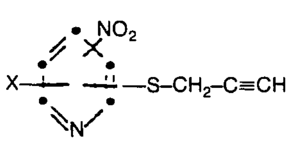

worin X Wasserstoff oder Halogen bedeutet. Verfahren zur Herstellung dieser Verbindungen durch Umsetzung eines Pyridinthiols mit einem Propargylhalogenid sowie diese enthaltende Mittel zur Verwendung in der Schädlingsbekämpfung, insbesondere als Ovizide zur Bekämpfung von fressenden Schadinsekten auf dem Gebiet des Pflanzenschutzes.

EP 0 022 748 A1

5-12432/+

Pyridinderivate

BEZEICHNUNG GEÄNDERT
siehe Titelseite

Die vorliegende Erfindung betrifft neue Propargylthio-nitropyridine, Verfahren zu ihrer Herstellung und ihre Verwendung in der Schädlingsbekämpfung.

Die erfindungsgemässen Propargylthio-nitropyridine haben die Formel I

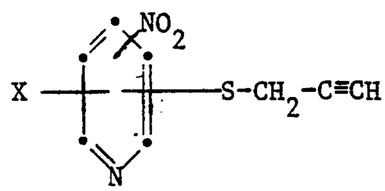

(I)

worin X Wasserstoff oder Halogen bedeutet.

Wegen ihrer Wirkung als Schädlingsbekämpfungsmittel bevorzugt sind erfindungsgemäss Verbindungen der Formel I, die dadurch gekennzeichnet sind, dass die Propargylthiogruppe in der 2-Stellung steht. Hervorzuheben sind ferner diejenigen Verbindungen der Formel I, worin X Wasserstoff oder Chlor bedeutet. Besonders wertvoll sind aufgrund ihrer biologischen Wirksamkeit solche Verbindungen der Formel I, worin die Nitrogruppe in der 3- oder 5-Stellung steht und X Wasserstoff bedeutet.

Die Verbindungen der Formel I können analog an sich bekannter Arbeitsweisen, wie sie für S-Alkylierungen von aromatischen und heteroaromatischen Thiolen gebräuchlich sind, hergestellt werden.

So kann man z.B. eine Verbindung der Formel I erhalten, durch Umsetzung eines Pyridinthiols der Formel II

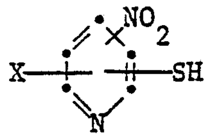

(II)

in Gegenwart eines Säureacceptors mit einem Propargylhalogenid der Formel III

$$Y-CH_2-C{\equiv}CH \qquad (III) \quad .$$

In den obigen Formeln II und III hat der Rest X die vorstehend unter Formel I angegebene Bedeutung und Y bedeutet Halogen, vorzugsweise Chlor oder Brom.

Das vorerwähnte Verfahren kann vorzugsweise unter normalem Druck und in Gegenwart eines organischen Lösungs- oder Verdünnungsmittels durchgeführt werden. Als Lösungs- oder Verdünnungsmittel eignen sich z.B. Alkohole, insbesondere niedere Alkohole, wie Methanol, Aethanol, Propanole usw.; Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische und aromatische Kohlenwasserstoffe, z.B. Benzol, Toluol und Xylol; Dimethylsulfoxid sowie Ketone, z.B. Aceton, Methyläthylketon, Methylisopropylketon und Methylisobutylketon. Das Verfahren wird im allgemeinen bei einer Temperatur von 0 bis 150° C, vorzugsweise zwischen 25 und 125° C, in Gegenwart eines Säureacceptors durchgeführt. Als Säureacceptoren können anorganische Basen, wie $K_2CO_3$, $Na_2CO_3$, $CaCO_3$ und entsprechende Hydrogencarbonate, oder organische Basen, z.B. tertiäre Amine, wie Triäthylamin, in Betracht kommen.

Die Ausgangsstoffe der Formeln II und III sind bekannt oder können analog bekannten Verfahren hergestellt werden. So werden z.B. Verbindungen der Formel II bzw. deren Herstellung in folgenden Literaturstellen beschrieben: J. Pharm. Soc. Jap. 64, 201 (1944); J.Am.Chem.Soc. 62, 1697 (1940); Yakugaku Zasshi 78, 417 (1958) und deutsche Patentschrift 1 014 375.

Aus der schweizerischen Patentschrift 528 210 ist die Verwendung ungesättigter Aether zur Bekämpfung von Ektoparasiten, wie z.B. Zecken, Milben und Vektoren, bekannt. Die dort aufgeführten substituierten Phenylpropargyläther sind jedoch zur Verwendung als Insektizide nur bedingt geeignet.

Ueberraschenderweise wurde nun gefunden, dass die erfindungsgemässen Verbindungen der Formel I bei guter Pflanzenverträglichkeit und geringer Warmblütertoxizität ausgezeichnete Wirksamkeit als Schädlingsbekämpfungsmittel, vor allem zur Bekämpfung von Pflanzen befallenden Insekten, aufweisen.

Beispielsweise eignen sich die Verbindungen der Formel I zur Bekämpfung von Insekten der Familien: Acrididae, Blattidae, Gryllidae, Gryllotalpidae, Tettigoniidae, Cimicidae, Phyrrhocoridae, Reduviidae, Aphididae, Delphacidae, Diaspididae, Pseudococcidae, Chrysomelidae, Coccinellidae, Bruchidae, Scarabaeidae, Dermestidae, Tenebrionidae, Curculionidae, Tineidae, Noctuidae, Lymantriidae, Pyralidae, Galleridae, Culicidae, Tipulidae, Stomoxydae, Muscidae, Calliphoridae, Trypetidae und Pulicidae.

Die Verbindungen der Formel I zeichnen sich vor allem durch eine breite ovizide und ovolarvizide Wirkung aus, die sich auf Eier und Eigelege von Schadinsekten erstreckt. Besonders hervorzuheben ist in diesem Zusammenhang die Wirksamkeit der erfindungsgemässen Verbindungen gegen Vertreter der Ordnung Lepidoptera, insbesondere der Familie Noctuidae. Daher können die Verbindungen der Formel I auch zur Bekämpfung von pflanzenschädigenden Frassinsekten, in Zier- und Nutzpflanzungen, insbesondere in Baumwollkulturen (z.B. gegen Spodoptera littoralis und Heliothis virescens) eingesetzt werden.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen.

Als Zusätze kommen z. B. folgende Wirkstoffe in Betracht: organische Phosphorverbindungen, Nitrophenole und Derivate, Pyrethroide, Formamidine, Harnstoffe, Carbamate und chlorierte Kohlenwasserstoffe.

Mit besonderem Vorteil kann man die Verbindungen der Formel I auch mit Substanzen kombinieren, welche einen pestizid verstärkenden Effekt ausüben. Beispiele solcher Verbindungen sind u.a.: Piperonylbutoxid, Propionyläther, Propionyloxime, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithionate.

Die Verbindungen der Formel I können für sich allein oder zusammen mit geeigneten Trägern und/oder Zuschlagstoffen eingesetzt werden. Geeignete Träger und Zuschlagstoffe können fest oder flüssig sein und entsprechen den in der Formulierungstechnik üblichen Stoffen, Lösungs-, Dispergier- , Netz-, Haft-, Verdickungs-, Binde- und/oder Düngemitteln. Zur Applikation können die Verbindungen der Formel I zu Stäubemitteln, Emulsionskonzentraten, Granulaten, Dispersionen, Sprays, zu Lösungen oder Aufschlämmungen in üblicher Formulierung, die in der Applikationstechnik zum Allgemeinwissen gehören, verarbeitet werden.

Die Herstellung erfindungsgemässer Mittel erfolgt in an sich bekannter Weise durch inniges Vermischen und/oder Vermahlen von Wirkstoffen der Formel I mit den geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden:

Feste Aufarbeitungsformen: Stäubemittel, Streumittel, Granulate (Umhüllungsgranulate, Imprägnierungsgranulate und Homogengranulate);

Flüssige Aufarbeitungsformen:

a) in Wasser dispergierbare Wirkstoffkonzentrate: Spritzpulver (wettable powders), Pasten, Emulsionen;

b) Lösungen.

Der Gehalt an Wirkstoff in den oben beschriebenen Mitteln liegt zwischen 0,1 bis 95 %.

Die Wirkstoffe der Formel I können beispielsweise wie folgt formuliert werden:

<u>Stäubemittel:</u> Zur Herstellung eines a) 5%igen und b) 2%igen Stäubemittels werden die folgenden Stoffe verwendet:

a) 5 Teile Wirkstoff,
95 Teile Talkum;

b) 2 Teile Wirkstoff,
1 Teil hochdisperse Kieselsäure,
97 Teile Talkum.

Die Wirkstoffe werden mit den Trägerstoffen vermischt und vermahlen.

<u>Granulat:</u> Zur Herstellung eines 5%igen Granulates werden die folgenden Stoffe verwendet:

5,00 Teile Wirkstoff,
0,25 Teile epoxydiertes Pflanzenöl,
0,25 Teile Cetylpolyglykoläther,
3,50 Teile Polyäthylenglykol,
91,00 Teile Kaolin (Korngrösse 0,3 - 0,8 mm).

Die Aktivsubstanz wird mit dem epoxydierten Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

Spritzpulver: Zur Herstellung eines a) 40%igen, b) und c) 25%igen d) 10%igen Spritzpulvers werden folgende Bestandteile verwendet:

a) 40 Teile Wirkstoff,
5 Teile Ligninsulfonsäure-Natriumsalz,
1 Teil Dibutylnaphthalinsulfonsäure-Natriumsalz,
54 Teile Kieselsäure;

b) 25,0 Teile Wirkstoff,
4,5 Teile Calcium-Ligninsulfonat,
1,9 Teile Champagne-Kreide/Hydroxyäthylcellulose-Gemisch (1:1),
1,5 Teile Natrium-dibutyl-naphthalinsulfonat,
19,5 Teile Kieselsäure,
19,5 Teile Champagne-Kreide,
28,1 Teile Kaolin;

c) 25,0 Teile Wirkstoff,
2,5 Teile Isooctylphenoxy-polyoxyäthylen-äthanol,
1,7 Teile Champagne-Kreide/Hydroxyäthylcellulose-Gemisch (1:1),
8,3 Teile Natriumaluminiumsilikat,
16,5 Teile Kieselgur,
46,0 Teile Kaolin;

d) 10 Teile Wirkstoff,
3 Teile Gemisch der Natriumsalze von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf entsprechenden Mühlen und Walzen

vermahlen. Man erhält Spritzpulver, die sich mit Wasser zu Suspensionen jeder gewünschten Konzentration verdünnen lassen.

Emulgierbare Konzentrate: Zur Herstellung eines a) 10%igen b) 25%igen und c) 50%igen emulgierbaren Konzentrates werden folgende Stoffe verwendet:

a) 10,0 Teile Wirkstoff,
3,4 Teile epoxydiertes Pflanzenöl,
3,4 Teile eines Kombinationsemulgators, bestehend aus Fettalkoholpolyglykoläther und Alkylaralkyl-sulfonat-Calcium-Salz,
40,0 Teile Dimethylformamid,
43,2 Teile Xylol;

b) 25,0 Teile Wirkstoff,
2,5 Teile epoxydiertes Pflanzenöl,
10,0 Teile eines Alkylarylsulfonat/Fettalkoholpolyglykoläther-Gemisches,
5,0 Teile Dimethylformamid,
57,5 Teile Xylol;

c) 50,0 Teile Wirkstoff,
4,2 Teile Tributylphenol-Polyglykoläther,
5,8 Teile Calcium-Dodecylbenzolsulfonat,
20,0 Teile Cyclohexanon,
20,0 Teile Xylol.

Aus solchen Konzentrationen können durch Verdünnen mit Wasser Emulsionen jeder gewünschten Konzentration hergestellt werden.

Sprühmittel: Zur Herstellung eines a) 5%igen und b) 95%igen Sprühmittels werden die folgenden Bestandteile verwendet:

0022748

a) 5 Teile Wirkstoff,
1 Teil epoxydiertes Pflanzenöl,
94 Teile Benzin (Siedegrenzen 160 bis 190° C);

b) 95 Teile Wirkstoff,
5 Teile epoxydiertes Pflanzenöl.

Beispiel 1: 7 g 5-Nitro-pyridin-2-thiol werden in Aceton suspendiert und mit 5 g Kaliumcarbonat versetzt. Darauf werden 6 g 3-Brompropin-1 zugefügt und, nachdem die exotherme Reaktion beendet ist, wird das Reaktionsgemisch etwa 30 Minuten am Rückfluss erhitzt. Man giesst die erhaltene Lösung auf Eis und filtriert den ausgefallenen Niederschlag ab. Die Reinigung des Rohproduktes erfolgt durch Umkristallisation aus Methanol. Man erhält 2-Propargylthio-5-nitropyridin vom Schmelzpunkt 98 bis 99,5° C.

Analog der vorstehend beschriebenen Arbeitsweise werden auch die folgenden unter Formel I fallenden Verbindungen hergestellt:

2-Propargylthio-3-nitropyridin Smp. 119 - 121° C
2-Propargylthio-3-nitro-5-chlorpyridin Smp. 82 - 84°C
2-Propargylthio-3-chlor-5-nitropyridin Smp. 143 - 146°C
2-Nitro-5-propargylthiopyridin .

Beispiel 2: Ovizide Wirkung auf Heliothis virescense und Spodoptera littoralis:

Entsprechende Mengenanteile einer benetzbaren pulverförmigen Formulierung, enthaltend 25 Gew.-% des zu prüfenden Wirkstoffes, wurden mit jeweils soviel Wasser verdünnt, dass sich wässrige Emulsionen von ansteigender Wirkstoffkonzentration ergaben.

In diese wirkstoffhaltigen Emulsionen wurden eintägige, auf Fliesspapier abgelegte Eigelege von Heliothis bzw. Spodoptera während drei Minuten eingetaucht und dann auf Rundfiltern abgenutscht. Die so behandelten Gelege wurden nach dem Antrocknen der Emulsion in Petrischalen ausgelegt und bei 28° C in der Dunkelheit aufbewahrt. Nach etwa 4 Tagen wurde die Schlupfrate im Vergleich zu unbehandelten Kontrolle festgestellt. Zur Auswertung wurde die zur 100%igen Abtötung der Eier erforderliche minimale Wirkstoffkonzentration bestimmt.

Die Verbindungen gemäss Beispiel 1 zeigten in diesem Test gute ovizide Wirkung gegen die geprüften Schädlinge.

Beispiel 3: Wirkung gegen Aëdes Aegypti:

Auf die Oberfläche von 150 ml Wasser, das sich in einem Behälter befindet, wurde so viel einer 0,1 %igen acetonischen Lösung des Wirkstoffes pipettiert, dass Konzentrationen von je 10,5 und 1 ppm erhalten wurden. Nach Verdunsten des Acetons wurde der Behälter mit 30 bis 40 2-tägigen Aëdés-Larven beschickt. Nach 1, 2 und 5 Tagen wurde die Mortalität geprüft.

Verbindungen gemäss Beispiel 1 zeigten in diesem Test gute Wirkung gegen Aëdes aegypti.

Beispiel 4: Wirkung auf Laspeyresia Pominella (Eier):

Abgelegte Eier von Laspeyresia pomonella, die nicht älter als 24 Stunden waren, wurden auf Filterpapier für 1 Minute in eine acetonisch-wässrige Lösung enthaltend 400 ppm des zu prüfenden Wirkstoffes eingetaucht. Nach dem Antrocknen der Lösung wurden die Eier in Petrischalen ausgelegt und bei einer Temperatur von 28°C belassen. Nach 6 Tagen wurde der prozentuale Schlupf aus den behandelten Eiern bewertet. Verbindungen gemäss Beispiel 1 zeigte gute Wirkung in obigem Test.

Patentansprüche

1. Verbindung der Formel I

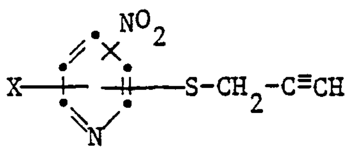

(I) ,

worin X Wasserstoff oder Halogen bedeutet.

2. Verbindung der Formel I gemäss Anspruch 1, dadurch gekennzeichnet, dass die Propargylthiogruppe in der 2-Stellung steht.

3. Verbindung der Formel I gemäss den Ansprüchen 1 oder 2, dadurch gekennzeichnet, dass X Wasserstoff oder Chlor bedeutet .

4. Verbindung der Formel I gemäss den Ansprüchen 1 oder 2, dadurch gekennzeichnet, dass die Nitrogruppe in der 3- oder 5-Stellung steht und X Wasserstoff bedeutet.

5. Verbindung gemäss Anspruch 3 der Formel

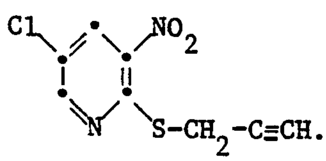

6. Verbindung gemäss Anspruch 4 der Formel

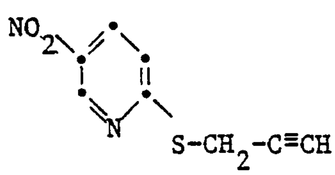

.

7. Verbindung gemäss Anspruch 4 der Formel

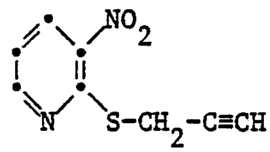

.

8. Verfahren zur Herstellung einer Verbindung gemäss den Ansprüchen 1 bis 7, dadurch gekennzeichnet, dass man eine Verbindung der Formel II

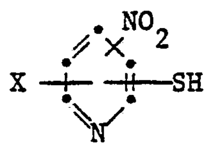

(II)

in Gegenwart eines Säureacceptors mit einer Verbindung der Formel III

$$Y—CH_2—C≡CH \quad (III)$$

umsetzt, wobei in Formel II der Rest X die in den Ansprüchen 1 und 3 angegebene Bedeutung hat und Y Halogen, vorzugsweise Chlor oder Brom, bedeutet.

9. Schädlingsbekämpfungsmittel, welches als aktive Komponente eine Verbindung gemäss den Ansprüchen 1 bis 7 zusammen mit geeigneten Trägern und/oder anderen Zuschlagstoffen enthält.

10. Verwendung einer Verbindung gemäss den Ansprüchen 1 bis 7 zur Bekämpfung von Schädlingen, vorzugsweise von Insekten.

11. Verwendung nach Anspruch 10 als Ovizid, vorzugsweise zur Bekämpfung von Insekten der Familie Noctuidae.

Europäisches Patentamt

# EUROPÄISCHER RECHERCHENBERICHT

Nummer der Anmeldung
0022748
EP 80 81 0220

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | CH - A - 528 210 (CIBA-GEIGY AG)<br>* Patentanspruch *<br>-- | 1,9 |
| A | US - A - 3 535 328 (ESSO RESEARCH AND ENGINEERING CO.)<br>* Verbindung 76 *<br>-- | |
| PA | US - A - 4 170 704 (GIVAUDAN CORP)<br>* Verbindung 10 *<br>---- | |

KLASSIFIKATION DER ANMELDUNG (Int. Cl.³)

A 01 N 43/40
C 07 D 213/70

RECHERCHIERTE SACHGEBIETE (Int. Cl.³)

C 07 D 213/70

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 26-09-1980 | VAN BIJLEN |